# EUROPÄISCHE PATENTANMELDUNG

(11) **EP 1 777 573 A1**
(43) Veröffentlichungstag der Anmeldung: **25.04.2007**
(21) Anmeldenummer: 06021582.9
(22) Anmeldetag: 14.10.2006
(51) Int. Cl.: G02B 23/24, G02B 6/04, A61B 1/07

(54) **Endoskop und Verfahren zu seiner Herstellung**

(30) Priorität: 18.10.2005 DE 102005051208
(71) Anmelder: Karl Storz GmbH & Co. KG, 78532 Tuttlingen (DE)
(72) Erfinder: Eisenkolb, Peter, 78532 Tuttlingen (DE)
(74) Vertreter: Weller, Wolfgang

(57) **Zusammenfassung**

Ein Endoskop (10), das einen Endoskopschaft (12) aufweist, der mit seinem proximalen Ende in einem Endoskopkopf (16) aufgenommen ist, weist einen Lichtleiteranschluss (18) auf. Im Endoskopkopf (16) ist ein Strang von Lichtleitern (20) vom Lichtleiteranschluss (18) über eine Krümmung (22) bis zu einem distalen Ende des Endoskopschaftes (12) geführt. Es wird vorgeschlagen, den Strang von Lichtleitern (20) im gekrümmten Bereich zwischen dem quer abstehenden Lichtleiteranschluss (18) und dem Endoskopschaft (12) durch Aufbringen eines flexiblen Klebstoffes (26) zu ummanteln, so dass einzelne Fasern (28) mit dem flexiblen Klebstoff (26) umhüllt sind (Fig. 2).

## Beschreibung

Die Erfindung betrifft ein Endoskop, mit einem Endoskopschaft, der an seinem proximalen Ende in einem Endoskopkopf aufgenommen ist, der einen Lichtleiteranschluss aufweist, wobei ein Strang von Lichtleitern vom quer abstehenden Lichtleiteranschluss über eine Krümmung zum Endoskopschaft und in diesem bis zu dessen distalen Ende geführt sind.

Die Erfindung betrifft ferner ein Verfahren zum Herstellen eines solchen Endoskops.

Endoskope der eingangs genannten Art sind allgemein bekannt.

Ein derartiges Endoskop findet seinen Einsatz am menschlichen Körper z.B. in der Arthroskopie, in der Bauch- und Brustkorbspiegelung, bei Leistenbrüchen sowie bei Gelenk- und Wirbelsäulenoperationen.

Endoskope der genannten Art weisen einen schlanken langgestreckten Endoskopschaft auf.

Dieser Endoskopschaft wird häufig durch zwei Rohre gebildet. Dabei sind ein äußeres Rohr und ein durchmessergeringeres inneres Rohr ineinander geschoben.

An seinem proximalen Ende ist der Endoskopschaft in einem Endoskopkopf aufgenommen.

Eines der Rohre weist als so genannte Optik ein Stablinsensystem oder ein modernes Bilderfassungssystem zur Bildwiedergabe auf.

Im Endoskopschaft ist zwischen dem äußeren und dem inneren Rohr ein Strang von Lichtleitern lose geführt. Dieser Strang weist häufig ein Bündel einzelner Fasern auf. Durch diese Lichtleitfasern wird im praktischen Einsatz das Operationsgebiet ausgeleuchtet.

Der Strang von Lichtleitern ist in dem Endoskopkopf in einem Lichtleiteranschluss gefasst. Dieser Lichtleiteranschluss steht quer zu dem Endoskopschaft gesehen von dem Endoskopkopf ab, so dass an ihm eine Lichtquelle angeschlossen werden kann. Von der angeschlossenen Lichtquelle wird Licht durch die Lichtleiter bis zum distalen Ende des Endoskopschaftes geschickt und tritt dort aus. Hierdurch wird das Operationsgebiet ausgeleuchtet.

Als Lichtleiter finden häufig z.B. gebündelte Quarzglasfasern ihren Einsatz. Die Lichtleiter können aber auch aus Mehrkomponentenglas oder Kunststoff hergestellt sein.

Im Endoskopkopf sind die Lichtleiter vom Lichtleiteranschluss über eine Krümmung zum Endoskopschaft und in diesem bis zu dessen distalen Ende geführt.

Dieser Bereich erweist sich im praktischen Einsatz als problematisch. Durch Krafteinwirkung kann es im Lauf des Gebrauchs zu feinen Bruchrissen im Endoskop kommen. Durch diese Risse kann Feuchtigkeit in den Endoskopkopf gelangen. Ferner können auf diesem Wege auch für eine Sterilisation verwendete chemisch aggressive Substanzen in den Endoskopkopf eintreten.

Hierdurch können die Lichtleiter während Autoklavierzyklen angegriffen und beschädigt werden. Dies erhöht besonders im gekrümmten Bereich im Endoskopkopf die Gefahr eines Faserbruchs und resultiert in einer stark verkürzten Gebrauchsdauer des Endoskops.

Aus der DE 102 22 686 A1 ist ein Endoskop bekannt, das zum Schutz eines Lichtleitfaserbündels eine Schutzhülle aufweist. Im Inneren der Schutzhülle ist ein Schmiermittel vorgesehen, das ein Silikonöl und ein festes Schmiermittel enthält. Das Lichtleitfaserbündel besteht aus mehreren Lichtleitfasern.

Beide Enden des Lichtleitfaserbündels sind mittels eines Haftmittels zu einem Bündel gebunden und an einem Anschlussteil bzw. einem distalen Endabschnitt des Endoskops befestigt. Das Schmiermittel dient einer Verringerung eines Biegewiderstandes, so dass längliche Elemente wie Lichtleiter vor einem Brechen bewahrt werden sollen. Ein derartiger Schutz ist in seiner Herstellung allerdings aufwendig.

Die DE 37 25 693 C2 offenbart ein Endoskop, das eine Leitfaser in Form eines Lichtleitfaserbündels aufweist. Ein Ende der Leitfaser ist mit einem distalen Ende eines Einführabschnittes verbunden und das andere Ende davon ist mit einem Bedienungsabschnitt verbunden.

Die Enden mehrerer Lichtleitfasern sind durch eine schmelzbare Masse miteinander verbunden.

Die Leitfaser weist in einem mittleren Bereich einen mäander- oder schleifenförmigen Abschnitt auf, der Belastungen aufnimmt, die entstehen, wenn der Einführabschnitt gebogen wird. Dabei kann der Abschnitt auch frei hängend sein.

Der mäanderförmige Bereich ist durch eine Schutzhülle umgeben. Einzelne Fasern sind nur an ihren Enden mit einem Überzug versehen. Zudem resultiert durch ein schleifenförmiges Führen des Lichtleitfaserbündels ein erhöhter Herstellungsaufwand.

Es ist daher Aufgabe der vorliegenden Erfindung, ein Endoskop der eingangs genannten Art dahingehend zu verbessern, dass es eine verlängerte Gebrauchsdauer bei gleichzeitig einfacher Herstellbarkeit aufweist.

Erfindungsgemäß wird die Aufgabe hinsichtlich des eingangs genannten Endoskopes dadurch gelöst, dass der Strang von Lichtleitern im Bereich der Krümmung zwischen dem Lichtleiteranschluss und dem Endoskopschaft durch Aufbringen eines flexiblen Klebstoffes ummantelt worden ist.

Hierdurch wird der Strang von Lichtleitern, der gekrümmt im Endoskopkopf angeordnet ist, vorteilhaft mit einer eng anliegenden äußeren Schutzhülle versehen. Diese Schutzhülle ist als ein Randbereich ausgebildet. Gleichzeitig bindet diese Schutzhülle auch einzelne Fasern des Stranges, wie in einem Verbundwerkstoff, untereinander.

Auf Grund der Verwendung des flexiblen Klebstoffes bleibt eine Biegsamkeit des Stranges erhalten.

Die Schutzhülle verstärkt den Strang dahingehend, dass dieser gegen eine mechanische Belastung besser geschützt ist. Mechanische Einflüsse, die von außen auf das Endoskop einwirken, wie z.B. Schläge und Stöße, werden durch eine Ummantelung gedämpft und somit aufgefangen. Dies schützt die Fasern vor einem Faserbruch.

Ferner gelangen Feuchtigkeit oder aggressive Chemikalien nicht mehr in diesem gekrümmten Bereich an die einzelnen Fasern. Dies verbessert den Schutz der Lichtleiter während eines Autoklavierens vor einem Faserbruch.

In einer weiteren bevorzugten Ausgestaltung ist der flexible Klebstoff feuchtigkeitsundurchlässig.

Diese Maßnahme hat den Vorteil, dass die Schutzhülle die Lichtleitfasern absolut vor Feuchtigkeit schützt.

In einer weiteren bevorzugten Ausgestaltung ist der flexible Klebstoff chemikalienbeständig.

Diese Maßnahme hat den Vorteil, dass in das Endoskop eintretende chemisch aggressive Substanzen durch den flexiblen Klebstoff von den Lichtleitern ferngehalten werden. Die Lichtleiter werden besonders während des Autoklavierens vor dem Faserbruch geschützt. Der Klebstoff dient somit als eine Barriere und trennt die Lichtleiter von Umwelteinflüssen ab.

In einer weiteren bevorzugten Ausgestaltung sind über den gekrümmten Bereich hinaus der Lichtleiteranschluss und der Eintrittsquerschnitt des Stranges im Endoskopschaft mit dem flexiblen Klebstoff versehen.

Diese Maßnahme hat den Vorteil, dass die Lichtleiter mit der Schutzhülle ummantelt sind und ferner zum einen der Lichtleiteranschluss und zum anderen der Eintrittsquerschnitt im Endoskopschaft so mit dem flexiblen Klebstoff versehen sind, dass diese beiden Eintrittsstellen im Endoskopkopf abgedichtet sind. Dies schließt die Möglichkeit aus, dass über diese beiden Eintrittsstellen in den Endoskopkopf Feuchtigkeit bzw. Chemikalien eindringt. Hierdurch sind die in der Krümmung liegenden Lichtleiter verbessert vor Feuchtigkeit und Chemikalien geschützt.

In einer weiteren bevorzugten Ausgestaltung sind die einzelnen Lichtleiter des Stranges ebenfalls mit dem flexiblen Klebstoff umhüllt.

Diese Maßnahme hat den Vorteil, dass nicht nur der Strang ummantelt ist, sondern auch die einzelnen Fasern des Stranges. Die mechanische Stabilität der einzelnen Fasern ist durch ein Umhüllen mit dem flexiblen Klebstoff, wie in einem Verbundwerkstoff verbessert. Ferner sind die einzelnen Fasern besser vor angreifenden Chemikalien oder vor Feuchtigkeit während des Autoklavierens geschützt. Die Wahrscheinlichkeit des Faserbruchs ist hierdurch stark vermindert.

Hinsichtlich des eingangs genannten Verfahrens zum Herstellen eines Endoskops, mit einem Endoskopschaft, der an seinem proximalen Ende in einem Endoskopkopf aufgenommen ist, bei dem von einem Lichtleiteranschluss zum Endoskopschaft ein Strang von Lichtleitern in einer Krümmung eingebracht werden und die Lichtleiter im Endoskopschaft bis zu dessen distalen Ende geführt sind, wird die Aufgabe dadurch gelöst, dass der Strang in der Krümmung durch Aufbringen eines flexiblen Klebstoffes ummantelt wird.

Das erfindungsgemäße Herstellungsverfahren hat den Vorteil, dass das Aufbringen des flexiblen Klebstoffes auf besonders einfache Weise durchführbar ist. Der flexible Klebstoff muss lediglich kurz vor einem endgültigen Zusammenbau des Endoskopkopfes auf die Lichtleiter im gekrümmten Bereich aufgebracht werden.

Besonders bevorzugt ist es, wenn der flexible Klebstoff in den Eintrittsquerschnitt des Stranges im Endoskopschaft eingebracht wird.

Diese Maßnahme ist vorteilhaft, da hierdurch der Endoskopschaft zum Endoskopkopf abgedichtet wird. Auf diese Weise kann vom Endoskopschaft keine Feuchtigkeit mehr in den Endoskopkopf eindringen. In Verbindung mit einer Abdichtung des Lichtleiteranschlusses ist ein Eindringen von Feuchtigkeit oder Chemikalien in den Endoskopkopf stark erschwert. Dies verbessert den Schutz der Lichtleiter im gekrümmten Bereich im Endoskopkopf vor solchen Substanzen.

In einer weiteren bevorzugten Ausgestaltung wird der flexible Klebstoff so auf die Lichtleiter aufgebracht, dass die einzelnen Lichtleiter ebenfalls mit dem flexiblen Klebstoff umhüllt werden.

Diese Maßnahme hat den Vorteil, dass sowohl die mechanische Belastbarkeit der einzelnen Fasern erhöht wird, als auch deren Resistenz gegen beschädigende Angriffe von Chemikalien oder Feuchtigkeit. Die Wahrscheinlichkeit des Faserbruchs ist hierdurch stark vermindert.

Es versteht sich, dass die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend an Hand eines ausgewählten Ausführungsbeispiels im Zusammenhang mit den beiliegenden Zeichnungen näher beschrieben und erläutert. Es zeigen:
- Fig. 1: eine perspektivische Ansicht eines erfindungsgemäßen Endoskopes;
- Fig. 2: einen Schnitt durch ein erfindungsgemäßes Endoskop;
- Fig. 3: eine schematische Skizze eines Schnittes entlang der Linie III-III aus Figur 2; und
- Fig. 4: ein Schnitt vergleichbar der Figur 3 durch einen Strang eines Lichtleiters mit vollständiger Durchtränkung mit Klebstoff.

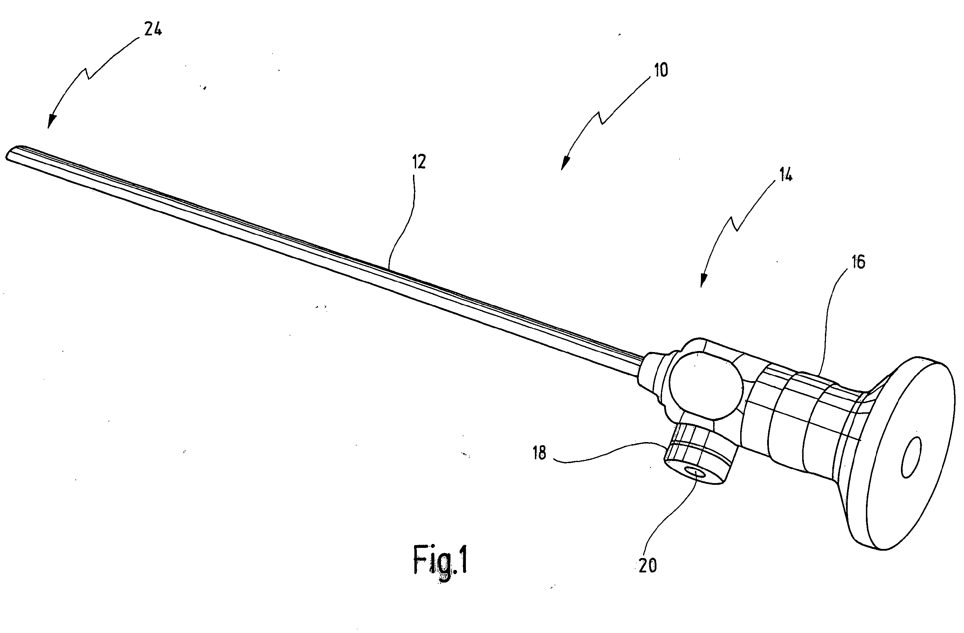

In Fig. 1 ist ein Endoskop dargestellt, das in seiner Gesamtheit mit der Bezugsziffer 10 bezeichnet ist.

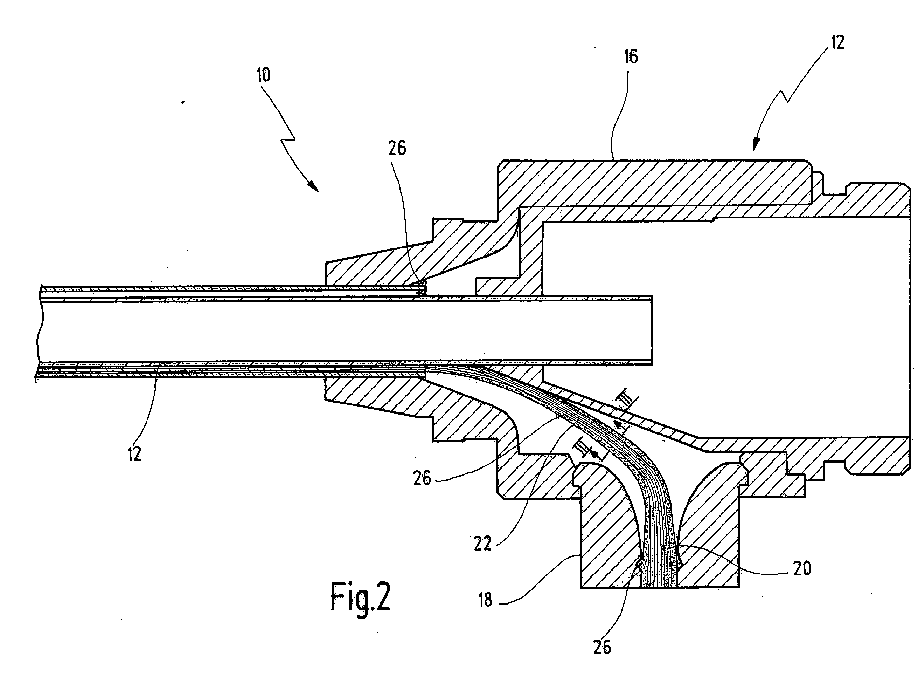

Das Endoskop 10 weist einen Endoskopschaft 12 auf. Der Endoskopschaft 12 ist an seinem proximalen Ende 14 in einem Endoskopkopf 16 aufgenommen. Dieser Endoskopkopf 16 weist einen quer abstehenden Lichtleiteranschluss 18 auf. In diesem Lichtleiteranschluss 18 sind, wie in Figur 2 dargestellt, Lichtleiter 20 proximal endend aufgenommen. Diese Lichtleiter 20 weisen die Form eines Bündels oder Stranges auf.

Figur 2 zeigt ferner, dass dieser Strang von Lichtleitern 20 vom quer abstehenden Lichtleiteranschluss 18 über eine Krümmung 22 zum Endoskopschaft 12 geführt ist.

Die Lichtleiter 20 erstrecken sich bis zum distalen Ende 24 des Endoskopschaftes 12.

Der Strang an Lichtleitern 20 ist im gekrümmten Bereich zwischen dem quer abstehenden Lichtleiteranschluss 18 und dem Endoskopschaft 12 mit einem flexiblen Klebstoff 26 ummantelt.

Dieser flexible Klebstoff 26 ist feuchtigkeitsundurchlässig und chemikalienbeständig.

Ferner sind sowohl die Mündungsöffnung des Lichtleiteranschlusses 18 und der Eintrittsquerschnitt des Stranges im Endoskopschaft 12 mit dem flexiblen Klebstoff 26 versehen und dadurch abgedichtet.

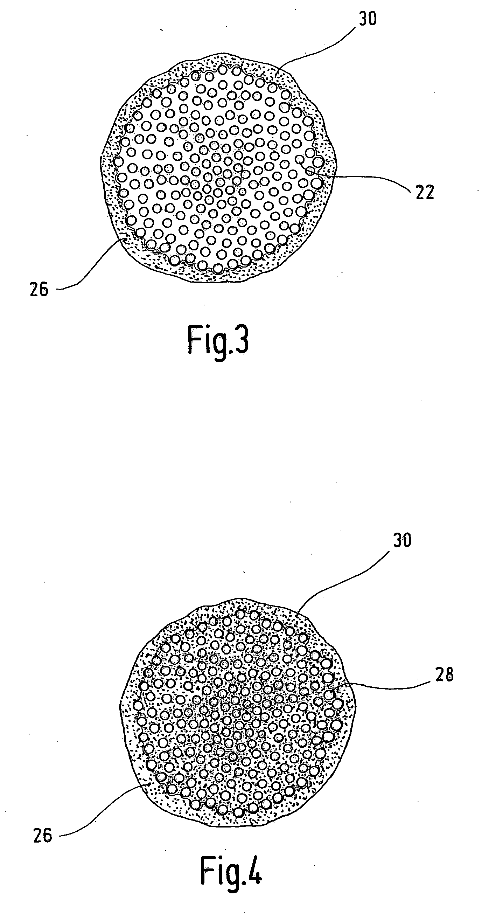

Figur 3 zeigt einen Schnitt entlang der Linie III-III der Figur 2. Darin ist veranschaulicht, dass der Strang von Lichtleitern 20 einzelne Fasern 28 aufweist. Der flexible Klebstoff 26 ist hierbei als eine äußere Schutzhülle 30 auf den Strang von einzelnen Fasern 28 aufgebracht. Diese Schutzhülle 30 ist enganliegend und dringt geringfügig in Zwischenräume zwischen die äußeren Fasern 28 ein. Die Fasern 28 im inneren des Stranges sind weiterhin aneinander vorbei bewegbar.

Figur 4 stellt einen vergleichbaren Schnitt wie in Figur 3 dar. Hierbei wird der flexible Klebstoff 26 alternativ so aufgebracht, dass der flexible Klebstoff 26 auch in die Zwischenräume zwischen die einzelnen Fasern 28 eindringt. Dies lässt sich beispielsweise durch Verwenden eines niedrigviskosen flexiblen Klebstoffes 26 erreichen. Hierdurch ist jede einzelne Faser 28 mit einer eigenen Ummantelung versehen.

Das Endoskop 10 wird wie üblich montiert.

Im Lichtleiteranschluss 18 wird der Strang von Lichtleitern 20 eingesetzt und beispielsweise durch ein Haftmittel fixiert. Der Strang von Lichtleitern 20 wird in den Endoskopschaft 12 bis zu dessen distales Ende 24 geführt. Dabei verläuft der Strang in einer Krümmung 22 im Endoskopkopf 16.

Durch ein Fenster im Endoskopkopf 16, das hier nicht dargestellt ist, kann nun noch der flexible Klebstoff 26 auf den Strang von Lichtleitern 20 aufgebracht werden. Dies kann durch Aufpinseln, Aufsprühen oder Tränken erreicht werden.

Hierbei wird der flexible Klebstoff 26 von dem Lichtleiteranschluss 18 über die Krümmung 22 bis in einen Eintrittsquerschnitt des Stranges im Endoskopschaft 12 eingebracht. Anschließend härtet der Klebstoff 26 aus.

Es wird darauf geachtet, dass Lichtleiter einzeln mit dem flexiblen Klebstoff 26 umhüllt werden. Abschließend wird das Fenster des Endoskopkopfes 16 verschlossen und das Endoskop somit fertiggestellt.

## Patentansprüche

1. Endoskop, mit einem Endoskopschaft (12), der an seinem proximalen Ende (14) in einem Endoskopkopf (16) aufgenommen ist, der einen Lichtleiteranschluss (18) aufweist, wobei ein Strang von Lichtleitern (20) vom quer abstehenden Lichtleiteranschluss (18) über eine Krümmung (22) zum Endoskopschaft (12) und in diesem bis zu dessen distalen Ende (24) geführt sind, **dadurch gekennzeichnet, dass** der Strang von Lichtleitern (20) im Bereich der Krümmung (22) durch Aufbringen eines flexiblen Klebstoffes (26) ummantelt worden ist.

2. Endoskop nach Anspruch 1, **dadurch gekennzeichnet, dass** der flexible Klebstoff (26) feuchtigkeitsundurchlässig ist.

3. Endoskop nach Anspruch 1 oder 2, **dadurch gekennzeichnet, dass** der flexible Klebstoff (26) chemikalienbeständig ist.

4. Endoskop nach einem der Ansprüche 1 bis 3, **dadurch gekennzeichnet, dass** über den gekrümmten Bereich hinaus der Lichtleiteranschluss (18) und der Eintrittsquerschnitt des Stranges im Endoskopschaft (12) mit dem flexiblen Klebstoff (26) versehen sind.

5. Endoskop nach einem der Ansprüche 1 bis 4, **dadurch gekennzeichnet, dass** die einzelnen Lichtleiter (20) des Stranges ebenfalls mit dem flexiblen Klebstoff (26) umhüllt sind.

6. Verfahren zur Herstellung eines Endoskopes, mit einem Endoskopschaft (12), der an seinem proximalen Ende (14) in einem Endoskopkopf (16) aufgenommen ist, bei dem von einem Lichtleiteranschluss (18) zum Endoskopschaft (12) ein Strang von Lichtleitern (20) in einer Krümmung (22) eingebracht werden und die Lichtleiter (20) im Endoskopschaft (12) bis zu dessen distalen Ende (24) geführt sind, **dadurch gekennzeichnet, dass** der Strang in der Krümmung (22) durch Aufbringen eines flexiblen Klebstoffes (26) ummantelt wird.

7. Verfahren nach Anspruch 6, **dadurch gekennzeichnet, dass** der flexible Klebstoff (26) in den Eintrittsquerschnitt des Stranges im Endoskopschaft (12) eingebracht wird.

8. Verfahren nach Anspruch 6 oder 7, **dadurch gekennzeichnet, dass** der flexible Klebstoff (26) so auf die Lichtleiter (20) aufgebracht wird, dass die einzelnen Lichtleiter ebenfalls mit dem flexiblen Klebstoff (26) umhüllt werden.
